(19) Europäisches Patentamt
European Patent Office
Office européen des brevets

(11) Publication number: **0 416 097 A1**

# (12) EUROPEAN PATENT APPLICATION published in accordance with Art. 158(3) EPC

(21) Application number: **88902939.3**

(22) Date of filing: **30.03.88**

(86) International application number:
**PCT/JP88/00325**

(87) International publication number:
**WO 89/09274 (05.10.89 89/24)**

(51) Int. Cl.[5]: **C12P 17/18**, C12N 5/00, //(C12P17/18,C12R1:91)

(43) Date of publication of application:
**13.03.91 Bulletin 91/11**

(84) Designated Contracting States:
**DE FR GB IT NL**

(71) Applicant: **SEITAIKINOU RIYOU KAGAKUHIN SHINSEIZOUGIJUTSU KENKYU KUMIAI**
**2-3, Marunouchi 2-chome Chiyoda-ku**
**Tokyo(JP)**

(72) Inventor: **DENO, Hiroshi**
**4-9, Wakicho Waki 2-chome Kuga-gun**
**Yamaguchi 740(JP)**
Inventor: **YAMAGATA, Hikaru**
**2-1, Muronokicho 1-chome Iwakuni-shi**
**Yamaguchi 740(JP)**
Inventor: **ITOH, Aya**
**2-6, Shozoku 3-chome**
**Iwakuni-shi Yamaguchi 740(JP)**
Inventor: **EMOTO, Tateki**

**deceased(JP)**

(74) Representative: **Myerscough, Philip Boyd et al**
**J.A.Kemp & Co. 14, South Square Gray's Inn**
**London, WC1R 5EU(GB)**

## (54) PROCESS FOR PRODUCING TROPANE ALKALOIDS.

(57) A process for producing tropane alkaloids in good yield, which comprises conducting tissue culture of tissue and/or cells of a plant yielding tropane alkaloids in a medium containing a compound or compounds selected from the group consisting of (A) amines, (B) sulfur-containing amino acids and/or hydroxyamino acids, (C) gibberellin, and (D) abscisic acid and/or anti-cytokinin substance.

EP 0 416 097 A1

# PROCESS FOR PRODUCING TROPANE ALKALOIDS

## Technical Field of the Invention

The present invention relates to a method for manufacturing tropane alkaloid such as scopolamine and/or hyoscyamine by means of tissue culture for plant tissue of such plants as Duboisia, Datura, Hyos, Japanese belladonna (Scopolia japonica maxim), etc. which metabolically produce tropane alkaloid by use of specific culture medium.

## Background of the Invention

Some alkaloids that are secondary metabolic products of plants are being used as important pharmaceuticals, for example, scopolamine as spasmolytic, analgesic and parasympatholytic drug; hyoscyamine as parasympatholytic drug. The above compounds are being manufactured by extraction from the body of natural plant. However, since the products of natural origin are used as raw materials of the compounds, the problems are that the production depends upon the weather and the period of harvest is limited. In view of the above, much study has been given to the production of the compounds by means of tissue culture of the plant. With regard to the production with callus, the production of Hyos with callus by Yamada et al. is already known. [Plant Cell Reports 1. 101 - 103 (1982)]. However, scopolamine content of 20 ppm obtained in the above study was low as compared with the content in the body of natural plant. In addition, Yamada et al. discovered the existence of considerable amounts of scopolamine and hyoscyamine in the adventitious root obtained by tissue culture of Duboisia Leichhardtii F. Muell [Plant Cell Reports 3, 186 - 188 (1984)]. Nevertheless, the content thus obtained was still insufficient. Their further investigation on a variety of culture conditions of the adventitious root of Duboisia led to the finding that the productivity of tropane alkaloid could be improved by setting the ratio of ammonium ions to nitrate ions in the culture medium to not less than 0.2 and maintaining the concentration of dissolved oxygen in the culture medium in the range of 10 to 65 ppm. As a result, they filed patent applications as Japanese patent applications No. sho-60 (1985)-143882 and No. sho-67 (1985)-143881. However, it is hoped that the productivity of tropane alkaloid be further improved from the industrial point of view.

Therefore, in the case that the tissue culture is directed toward the industrial production of tropane alkaloid, further improvement on the productivity of the alkaloid has been an important subject.

In view of the above, the inventors have made intensive study on the method for effectively culturing the tissues and cells of the adventitious root, etc. of the plants which produce tropane alkaloid such as Duboisia, Datura, Hyos, Japanese belladonna, etc. As a reult, the inventors have discovered the fact described hereunder.

## Disclosure of the Invention

The inventors have discovered that when the tissues or cells of the above-mentioned plants are subjected to tissue culture by use of a culture medium containing at least one specific compound, the content or tropane alkaloid in the tissues or cells thus obtained is improved, or the growth of the tissues and cells of the adventitious root, etc. is accelerated, each resulting in the improvement on the productivity of tropane alkaloid.

Further research and investigation on the basis of the above knowledge finally completed the present invention.

By the method of the present invention is provided the method for manufacturing tropane alkaloid characterized in that tropane alkaloid is manufactured by subjecting the tissues or cells of the plants which produce tropane alkaloid to tissue culture by use of a culture medium containing at least one compound selected from the group consisting of (A) amines; (B) sulfur-containing amino acids and/or oxyamino acids; (C) gibberellin; and (D) abscisic acid and/or anti-cytokinin substances.

## Best Mode of Carrying Out the Invention

The plants to which the method of the present invention is applicable are specifically exemplified by the

Solanaceous plants including the plants of the genus Duboisia such as Duboisia myoporoids, Duboisia leichharatii, etc., those of the genus Datura such as Datura tatula, Datura arborea, Datura stramonium, etc., those of the genus Scopolia such as Scopolia japonica, etc., those of the genus Hyos such as Hyoscyamus niger, etc., and those of the genus Atropa such as Atropa belladonna, etc.

The method of the present invention is characterized by the use of at least one compound selected from the group consisting of (A) amines; (B) sulfur-containing amino acid and/or oxyamino acid; (C) gibberellin; and (D) abscisic acid and/or anticytokinin substances in the conventional culture medium which is being used for the tissue culture of plants for the purpose of subjecting the tissues or cells of the above-mentioned plant to tissue culture. In addition to the above compounds, inorganic ingredient and carbon source are essential, and plant hormones, vitamins, and when necessary, amino acids are added.

Examples of amines that can be used in the present invention are tetramethylenediamine and derivatives thereof such aş spermine [$H_2N-(CH_2)_3NH-(Ch_2)_4-NH-(CH_2)_3-NH_2$], spermidine [$H_2N-(CH_2)_3-NH-(CH_2)_4-NH_2$], putrescine [$H_2N-(CH_2)_4-NH_2$], etc. and polyalkylene polyamines and derivatives thereof such as diamino propane [$H_2N-(CH_2)_3-NH_2$], diaminopropyl amine [$H_2N-(CH_2)_3-NH-(CH_2)_3-NH_2$], etc.

The usage of amines in the present invention is adjusted to the concentration of said amines in the culture medium usually in the range of 0.01 mM to 10 mM, and preferably 0.1 mM to 5 mM. One or more amines from among the above-mentioned amines can be used in the present invention. The tissue culture of the above plant using the culture medium containing at least one amine according to the present invention can increase the yield of tropane alkaloid as compared with the culture medium not containing any of the above amines.

Sulfur-containing amino acids that can be used in the present invention are amino acids containing sulfur or derivatives thereof selected from methionines, cystines and cysteines, the detail of which is described hereunder: Methionines relating to the present invention are amino acids represented by the general formula [I]

$$\begin{array}{c} \quad\quad\quad\quad\quad NHR^1 \\ \quad\quad\quad\quad\quad | \\ R^2SCH_2CH_2CHCOR^3 \end{array} \qquad [I]$$

wherein $R^1$ is H or $R-\overset{\overset{O}{\|}}{C}$ (R is H or lower alkyl group), $R_2$ is H, lower alkyl group or L-alanyl group, and $R^3$ is OH, $NH_2$ or OH (M is metallic ion) and derivatives thereof, and specifically exemplified by methionine, N-formyl methionine, N-acetyl methionine, N-acylmethionines such as N-propyonyl methionine, etc., homocysteine, 2-amino-4-ethylthiobutanoic acid, 2-amino--4-alkyl-thiobutanoic acid except methionine such as 2-amino-4-propylthiobutanoic acid, cystathionine, 2-acylamino-4-alkylthio-butanoic acid except N-acyl methionine such as 2-acetyl-amino-4-ethylthiobutanoic acid, etc., 2-amino-4-alkylthiobutanoic acid amide such as 2-amino-4-methyl-thiobutanoic acid amide, etc., and 2-acylamino-4-alkyl-thiobutanoic acid amide such as 2-acetylamino-4-methyl-thiobutanoic acid amide, etc. In the present invention, the carboxyl groups in the above-mentioned compounds may form salts together with the same metallic ions as inorganic ingredients as described hereunder which constitute culture medium. Such salts can be used as the ingredients in the culture medium.

Cystines relating to the present invention are amino acids represented by the general formula [II]

$$\left( \begin{array}{c} \quad\quad\quad NHR^1 \\ \quad\quad\quad | \\ -SCH_2CHCOR^3 \end{array} \right)_2 \qquad [II]$$

wherein $R^1$ and $R^3$ are same as $R^1$ and $R^3$ in the general formula [I], respectively, and derivatives thereof, and specifically exemplified by cystine, N, $N^1$-diformyl cystine, N-$N^1$-diacetyl cystine, N, $N^1$-diacyl cystine such as N, $N^1$-dipropyonyl cystine, etc., bis (2-amino-2-aminocarbonylethyl) disulfide, bis (2-acylamino-2-aminocarbonylethyl) disulfide such as bis (2-acetylamino-2-aminocarbonylethyl) disulfide, etc.

In the present invention, the carboxyl groups in the above-mentioned compounds may form salts together with the same metallic ions as inorganic ingredients as described hereunder which constitute culture medium. Such salts can be used as the ingredients in the culture medium. Cysteines relating to the present invention are amino acids represented by the general formula [III];

$$-HSCH_2\underset{|}{\overset{NHR^1}{}}CHCOR^3 \qquad [III]$$

wherein $R^1$ and $R^3$ are same as $R^1$ and $R^3$ in the general formula [I], respectively, and derivatives thereof, and specifically exemplified by cysteine, N-formyl cysteine, N-acetyl cystein, N-acyl cystein such as N-propyonyl cysteine, and 2-acylamino-3-mercaptobutanoic acid amide such as 2-amino-3-mercaptobutanoic acid amide, 2-acetylamino-3-mercaptobutanoic acid amide, etc. In the present invention, the carboxyl groups in the foregoing compounds may form salts together with the same metallic ions as inorganic ingredients as described hereunder which constitute culture medium. Such salts are available as the ingredients in the culture medium.

In the tissue culture according to the present invention, aside from the aforementioned sulfur-containing amino acids, the oxyamino acids as mentioned above can be used alone or in combination with the sulfur-containing amino acids as indispensable ingreidents for the culture medium.

In this case, the oxyamino acids are amino acids having the general formula [IV];

$$R^4-CH(OH)\underset{|}{\overset{NHR^1}{}}CHCOR^3 \qquad [IV]$$

wherein $R^1$ and $R^3$ are as previously defined in the foregoing general formula [I] , and $R^4$ is H or $CH_3$, and derivatives thereof, and specifically exemplified by serine ($R^1$, $R^3$, $R^4$ = H), threonine ($R^1$, $R^3$ = H, $R^4$ - $CH_3$), N-formyl serine, N-acetyl serine, N-acyl serine such as N-propionyl serine, 2-amino-3-hydroxypropanoic-acid amide, 2-acylamino-3-hydroxypropanoic-acid amide such as 2-acetylamino-3-hydroxypropanoic-acid amide N-formyl threonine, N-acetyl threonine, N-acyl threonines such as N-propyonyl threonine, 2-aminobutanoic-acid amide, and 2-acylamino-butanoic acid amides such as 2-acetylaminobutanoic acid amide. In the present invention, the carboxyl groups in the aforestated compounds may form salts together with the same metallic ions as inorganic ingredients as described below which constitute culture medium. Such salts are available as the ingredients in the culture medium.

The usage of the above-mentioned sulfur-containing amino acids and/or oxyamino acids to be contained in the culture medium according to the present invention is adjusted to the concentration of said amino acids in the medium usually in the range of 0.3 mM to 10 mM, and preferably 0.5 to 3 mM. Where the combined use of the sulfur-containing amino acids and oxyamino acids is applied, the total amount of both ingredients is adjusted to the foregoing range in tissue culture. Since the use of the above acids departing from said range in the culture medium is not so effective in improving the yield of tropane alkaloid, the tissue culture according to the present invention is carried out by use of the culture medium containing the acid ingredient in said range.

Anticytokinin substances that can be used for the purpose of the present invention are, for example, the compounds having anticytokinin activity such as adenine, pyrido-pyrimidine, triazine derivatives, etc. The usage of abscisic acid is preferably adjusted so as to give the concentration of said acid in the range of $10^{-8}$ to $10^{-5}$M in the culture medium with the concentration of anticytokinin substances ranging from $10^{-8}$ to $10^{-5}$M.

Gibberellin that can be used in the present invention generically designates plant hormones having gibbern nucleus/es as expressed by the formula (1)

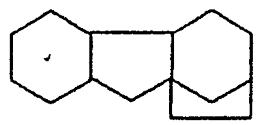

(1)

About 50 types of gibberellins (usually represented by GAn wherein n is an integer from 1 to 50, both inclusive) have been reported, each being applicable to the method of the present invention. The preferable gibberellin among a lot of types is gibberellin $A_3$($GA_3$) as expressed by the formula (2), which can increase the content of isoquinoline alkaloid such as berberine.

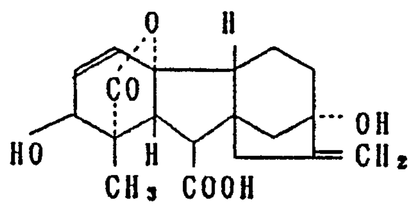

(2)

Preferable usage of gibberellin in the culture medium is usually at least $10^{-9}$ mol/ , and more preferably in the range of $10^{-6}$ to $10^{-3}$ mol/ℓ.

The method of tissue culture by use of the culture medium containing gibberellin is characterized not only by increase in the yield of tropane alkaloid, but also by marked increase in the exocytosis amount of the secondary metabolites. The above fact, therefore, enables the separation and recovery of tropane alkaloid from the culture system when compared with the conventional method, and also the repeated use of cultured product without discarding, resulting in improvement on the yield of tropane alkaloid.

Aside from the compound selected from the group consisting of (A) amines; (B) sulfur-containing amino acids and/or oxyamino acid; (C) gibberellin; and (D) abscisic acid and/or anticytokinin substances as described hereinbefore, inorganic ingredients to be contained in the culture medium used in the present invention are exemplified by the inorganic salt containing such element as nitrogen, phosphorus, potassium, sodium, calcium, magnesium, sulfur, iron, manganese, zinc, boron, molybdenum, chlorine, iodine, cobalt, etc., and more specifically by such compound as potassium nitrate, sodium nitrate, ammonium nitrate, ammonium chloride, potassium chloride, calcium chloride, potassium monohydrogenphosphate, potassium dihydrogenphosphate, magnesium sulfate, magnesium chloride, sodium sulfate, ferrous sulfate, ferric sulfate, manganese sulfate, copper sulfate, sodium molybdate, molybdenum trioxide, potassium iodide, zinc sulfate, boric acid, cobalt chloride, etc.

Examples of carbon source for the culture medium are carbohydrate of cane sugar and derivatives thereof, organic acids such as fatty acids, primary alcohols such as ethanol.

Plant hormones for the culture medium are, for example, naphthaleneacetic acid (NAA), indoleacetic acid (IAA), p-chlorophenoxyacetic acid, 2,4-dichlorophenoxyacetic acid (2, 4-D), indolebutyric acid (IBA), auxins and benzyladenines (BA) that are the derivatives, thereof cytokinins such as kinetin, zeatin, etc. It is preferable not to add cytokinins to the culture medium in the present invention. However, when cytokinin is added as required, it is preferably used at a lower concentration of $10^{-7}$M (0.02 mg/ℓ) at the maximum in the culture medium. Vitamins to be used in the culture medium are exemplified by biotin, thiamine (vitamin B), pyridoxine (vitamin B6), pyridoxel, pyridoxamine, calcium pantothenate, ascorbic acid (vitamin C), inositol, nicotinamide, riboflavin (vitamin B2), etc.

Examples of amino acids in the culture medium are glycin, alanine, glutamic acid, cysteine, phenylalanine, lysine, etc.

It is preferable that the culture medium according to the present invention usually contains about 0.1 μM to about 100 mM said inorganic ingredient, about 1g/ℓ to about 100 g/ℓ said carbon source, about 0.01 μM to about 100 μM said plant hormones, about 0.1 mg/ℓ to about 150 mg/ℓ said vitamins, and 0 to about 1,000 mg/ℓ said amino acids. The culture medium used for tissue culture of the plants of the genus Duboisia in the present invention is specifically exemplified by Murashige S. Skoog culture-medium ('62), Linsmaier & Skoog culture-medium (RM-1965), White culture-medium ('63), Gamborg B-5 culture-medium, Nitsch Nitsch culture-medium, etc., each being conventional culture medium being used for tissue culture of the plants but being added by the above-mentioned carbon source and plant hormones and, when

necessary, further added by the foregoing vitamins or amino acids. However, particularly preferable culture medium for the purpose of the present invention is that prepared by use of Nitsch Nitsch, Linsmaier & Skoog or Murashige & Skoog culture medium. The compositions of the above-mentioned conventional culture medium are described in "New tissue culture of plants" by Takeuchi, Nakajima, Furuya, pp. 386-391, 1979, published from Asakura Shoboh.

The culture medium applicable to the present invention is liquid culture-medium or solid culture-medium which contains 0.5 to 1.0% of agar-agar or gelatin. However, liquid culture medium is preferable for the present invention.

In the tissue culture according to the present invention, the tissue slices of a plant capable of metabolically producing tropane alkaloid are subjected to tissue culture using the afore-mentioned culture medium to obtain cultured cell or tissue each containing tropane alkaloid. The preferable tissue to be cultured in the present invention is adventitious root.

The tissue of the aforestated plants to be used for tissue culture according to the present invention is specifically exemplified by the cultured cell or tissue of the plant obtained by tissue culture of the method of the present invention or that of conventional method as well as roots, leaves, stems, seeds, buds, etc. of the plants. It is particularly preferable in the present invention to subject adventitious roots obtained by preliminary tissue culture of the plant tissue to tissue culture by use of the culture medium according to the present invention. In this case, the adventitious roots as raw material are proliferously cultured in the culture medium according to the present invention to produce adventitious roots containing a large amount of tropane alkaloid.

It is preferable in the present invention to apply the method of tissue culture wherein callus is derived from the above-mentioned tissue slices or cells and subcultured to produce cultured cells or tissues, which is then proliferously cultured by use of the culture medium according to the present invention to obtain cultured product, especially adventitious roots containing a large amount of tropane alkaloid.

In the case that adventitious roots are used in the present invention, the tissue slices of a plant can be infected with, for example, hair root disease bacteria (e.g. Agrobacterium rhizogenes) to generate hair roots, which can be used in the tissue culture. (For example, the method proposed in relation to the applicant in the patent application No. sho-61 (1986)-89975 can be applied.)

Tropane alkaloid obtained by the method of the present invention is exemplified by scopolamine, hyoscyamine and acetyl compounds of these compounds, but the preferable ones are scopolamine and hyoscyamine.

With regard to the method of separating tropane alkaloid from the cultured cells containing the same in the present invention, the conventional method, for example, described in the Pharmacopeia which has been used for isolating and purifying tropane alkaloid from the plant containing the above compound can be adopted.

The examples which follow specifically illustrate the method of the present invention in more detail in comparison with the comparative examples.

Example 1.

The leaves of Duboisia myporoides B. Br cultivated in the herb garden of the present applicant are washed, immersed in 10% antiformin liquid for 10 minutes, washed with sterilized water 3 times, cut into pieces of about 1 cm, implanted in agar-agar culture medium of Linsmaier & Skoog added by naphthaleneacetic acid and benzyladenine so as to cause concentrations of $10^{-5}$M and $10^{-6}$M, respectively, and cultured at 25°C for 30 days. The adventitious roots generated simultaneously with callus formation are collected by cutting, implanted in Nitsch Nitsch liquid culture-medium added by indolebutyric acid so as to cause a concentration of $10^{-5}$M, and subcultured for 2 years. The adventitious roots of 10 mg (dry weight) thus obtained is added by spermidine to a concentration of 1 mM, transplanted in Nitsch Nitsch liquid culture-medium added by indolebutyric acid so as to cause a concentration of $10^{-5}$M, said medium of 20 mℓ being placed in a 100 m Erlenmeyer flask, and cultured with shaking for 3 weeks. The adventitious roots of 190 mg (dry weight) thus obtained, after being dried, is extracted with 50 mℓ solution of basic chloroform-methanol. The extract thus obtained is added by 1 $NH_2SO_4$ (sulfuric acid) of 40 mℓ, and the alkaloid layer is transferred to $H_2SO_4$ layer. The alkaloid-containing layer is transferred to chloroform layer by adding 2 mℓ ammonia water and 40 mℓ chloroform, concentrated under reduced pressure, and analyzed by gas chromatography for the content of alkaloid. The yield of alkaloid is shown in Table 1. The analysis by gas chromatography is carried out under the following condition:

| | |
|---|---|
| Column: | Silicon OV-17 (1%) on Chromosorb W (Mesh 80-100) 3 mm diameter x 1 M glass column |
| Carrier gas : | $N_2$ |
| Column temperature: | 200 ° C |

Examples 2 - 6 and Comparative example 1

Example 1 is repeated with amines including putrescine, diamino propane, diamino ethane, cadaverine, or diamino propane to be added to the culture medium instead of spermidine. The results (Examples 2 - 6) are listed in Table 1, together with the comparative example 1 in which the same procedure as that in Example 1 is applied except that no amine is added.

Example 7

The leaves of Duboisia myporoides B. Br are treated with 10% antiformin and implanted in agar-agar culture medium of Linsmaier & Skoog (LS) containing benzyladenine in a concentration of $10^{-5}$M. The shoots which grow after one month are subcultured in the same culture medium for 40 days. The unlignified stems of the Duboisia shoots thus obtained are cut into the pieces of 1 - 2 cm in size, immersed in the suspension of Agrobacterium rhizogenes HRI-1 ($10^7$ nos./mℓ) which has been cultured with shaking for 24 hours, and then implanted in agar-agar LS culture medium not containing plant hormone. As a result, adventitious roots originate from the cut portion of the stems of the shoots after 3 weeks, and are treated in LS liquid culture-medium containing 0.1 mg/mℓ of ampicillin for 2 days to obtain self-proliferative Duboisia-infected adventitious roots, which are then subcultured in the LS liquid culture-medium for one (1) year. The adventitious roots of 10 mg (dry weight) thus obtained are transplanted in 20 mℓ of LS liquid culture-medium added by spermidine so as to cause a concentration of 1 mM, said medium being placed in a 100 mℓ Erlenmeyer flask, and cultured with shaking for 2 weeks. The adventitious roots of 150 mg (dry weight) thus obtained, after being dried, are extracted in the same manner as Example 1. The extract is analyzed for the yield of alkaloid. The result is listed in Table 2.

Example 8

Example 7 is repeated with putrescine as amines to be added to the culture medium instead of spermidine. The result is exhibited in Table 2.

Comparative example 2

The result of comparative example 2 in which the same procedure as that in Example 7 is followed except that no spermidine is added to the culture medium is shown in Table 2.

Table 1

| Experiment | Substance added | Amount of addition (mM) | Amount of growth (g/ℓ) | Scopolamine (mg/ℓ) | Hyoscyamine (mg/ℓ) |
|---|---|---|---|---|---|
| Comparative example 1 | --- | 0 | 7.5 | 34.4 | 10.3 |
| Example 1 | Spermidine | 1 | 9.5 | 91.2 | 30.3 |
| Example 2 | Putrescine | 3 | 8.6 | 82.6 | 27.1 |
| Example 3 | Diaminopropane | 1 | 7.6 | 76.8 | 25.6 |
| Example 4 | Diaminoethane | 0.5 | 8.0 | 72.0 | 26.5 |
| Example 5 | Cadaverine | 0.1 | 6.0 | 66.0 | 20.3 |
| Example 6 | Diaminopropylamine | 0.1 | 6.5 | 69.0 | 25.2 |

Table 2

| Experiment | Substance added | Amount of addition (mM) | Amount of growth (g/ℓ) | Scopolamine (mg/ℓ) | Hyoscyamine (mg/ℓ) |
|---|---|---|---|---|---|
| Comparative example 1 | --- | 0 | 4.1 | 10.7 | 29.5 |
| Example 7 | Spermidine | 1 | 7.5 | 16.5 | 63.8 |
| Example 8 | Putrescine | 1 | 7.3 | 16.1 | 54.8 |

Culture tissue ... Used by means of infection with hair root disease bacteria

Examples 9, 10, 12 and 13

The adventitious roots of 10 mg (dry weight) obtained by two years subculture in the same manner as Example 1 are transplanted in 20 mℓ or Nitsch Nitsch liquid culture-medium added by indolebutyric acid so as to cause a concentration of $10^{-5}$M, said medium being placed in a 100 mℓ Erlenmeyer flask, and cultured for 10 days. Thereafter, L-methionine is added to said liquid culture-medium to concentrations of 0.3 mM, 0.5 mM, 1,0 mM and 2.0 mM, respectively, followed by further culturing with shaking for 11 days. The adventitious roots thus obtained are extracted in the same manner as Example 1. The extract is analyzed for the yield of alkaloid. The results are indicated in Table 3.

Example 11

The adventitious roots of 10 mg (dry weight) obtained by two years subculture in the same manner as Example 1 are transplanted in 20 mℓ or Nitsch Nitsch liquid culture-medium added by L-methionine and indolebutyric acid to concentrations of 1.0 mM and $10^{-5}$M, respectively, said medium being placed in a 100 mℓ Erlenmeyer flask, and cultured with shaking for 3 weeks. The adventitious roots thus obtained, after being dried, are extracted in the same manner as Example 1. The extract is analyzed for the yield of alkaloid, the result of which is also listed in Table 3.

Comparative example 3

The result of comparative example 3 in which the same procedure as that in Example 9 is applied except that no L-methionine is added to the culture medium is also shown in Table 3.

Comparative example 4

Example 9 is repeated with L-methionine in a concentration of 0.2 mM instead of 0.3 mM. The result is also listed in Table 3.

Examples 14 and 15

Example 11 is repeated with L-cystine in concentrations of 0.5 mM and 1.0 mM, respectively instead of L-methionine. The results are also exhibited in Table 3.

Comparative examples 5 and 6

Example 11 is repeated with L-cystine in concentrations of 0.1 mM and 0.2 mM, respectively instead of L-methionine. The results are also indicated in Table 3.

Examples 16, 17 and 18

Example 11 is repeated with L-cysteine in concentrations of 0.3 mM, 0.5 mM, and 3 mM, respectively instead of L-methionine. The results are also shown in Table 3.

Comparative example 7

Example 11 is repeated with 0.1 mM l-cysteine instead of L-methionine. The result is also listed in Table 3.

Example 19 and 20

Example 11 is repeated with L-serine in concentrations of 0.5 mM and 3.0 mM, respectively instead of L-methionine. The results are also exhibited in Table 3.

Comparative example 8

Example 11 is repeated with 0.1 mM L-serine instead of L-methionine. The result is also indicated in Table 3.

Comparative examples 9 - 14

Example 9 is repeated with 1.0 mM amino-acids as listed in Table 1 instead of L-methionine. The results are also shown in Table 3.

Table 3

| Experiment | Substance added | Amount of addition (mM) | Time of addition | Amount of growth (g/ℓ) | Scopolamine (mg/ℓ) | Hyoscyamine (mg/ℓ) |
|---|---|---|---|---|---|---|
| Comparative example 3 | --- | 0 | --- | 7.5 | 34.4 | 10.3 |
| Comparative example 4 | L-methiomine | 0.2 | 10 days after the start of culture | 7.5 | 39.0 | 14.5 |
| Example 9 | - do - | 0.3 | " | 7.4 | 56.0 | 20.0 |
| " 10 | - do - | 0.5 | " | 7.6 | 73.0 | 24.3 |
| " 11 | - do - | 1.0 | Initial stage of culture | 6.0 | 60.6 | 19.5 |
| " 12 | - do - | 1.0 | 10 days after the start of culture | 7.5 | 75.8 | 26.1 |
| " 13 | - do - | 2.0 | " | 6.5 | 59.8 | 26.0 |
| Comparative example 5 | L-cystine | 1.0 | Initial stage of culture | 7.5 | 35.0 | 10.5 |
| Comparative example 6 | - do - | 0.2 | " | 7.5 | 35.0 | 11.0 |
| Example 14 | - do - | 0.5 | " | 7.5 | 50.0 | 19.5 |
| Example 15 | - do - | 1.0 | " | 7.6 | 59.0 | 20.5 |
| Comparative example 7 | L-cysteine | 0.1 | " | 7.5 | 37.5 | 13.1 |
| Example 16 | - do - | 0.3 | " | 7.5 | 50.5 | 17.5 |
| Example 17 | - do - | 0.5 | " | 7.6 | 56.5 | 19.0 |
| Example 18 | - do - | 3.0 | " | 7.3 | 60.5 | 21.0 |
| Comparative example 8 | L-serine | 0.1 | " | 7.5 | 34.0 | 11.5 |
| Example 19 | - do - | 0.5 | " | 7.3 | 50.5 | 18.3 |
| Example 20 | - do - | 3.0 | " | 7.2 | 60.3 | 20.8 |
| Comparative example 9 | L-aspartic acid | 1.0 | 10 days after the start of culture | 6.0 | 32.0 | 10.5 |
| Comparative example 10 | L-asparagine | 1.0 | " | 7.8 | 36.5 | 12.4 |
| Comparative example 11 | L-glutamic acid | 1.0 | " | 5.4 | 30.4 | 9.8 |
| Comparative example 12 | L-glutamine | 1.0 | " | 5.0 | 31.2 | 10.5 |
| Comparative example 13 | L-leucine | 1.0 | " | 6.0 | 34.0 | 11.8 |
| Comparative example 14 | L-valine | 1.0 | " | 5.0 | 25.0 | 9.5 |

Example 21

Example 7 is repeated with L-methionine instead of spermidine. The result is exhibited in Table 4.

Example 22

Example 21 is repeated with L-serine in a concentration of 3 mM in the culture medium in stead of L-methionine. The result is indicated in Table 4.

Comparative example 15

Example 21 is repeated without the addition of L-methionine. The result is also shown in Table 4.

Table 4

| Experiment | Substance added | Amount of addition (mM) | Amount of growth (g/ℓ) | Scopolamine (mg/ℓ) | Hyoscyamine (mg/ℓ) |
|---|---|---|---|---|---|
| Comparative example 15 | --- | 0 | 4.1 | 10.7 | 29.5 |
| Example 21 | L-methionine | 1.0 | 6.0 | 19.8 | 66.0 |
| Example 22 | L-serine | 3.0 | 6.0 | 18.5 | 58.1 |

Example 23

Example 1 is repeated by use or gibberellin $GA_3$ in $10^{-5}$M instead of spermidine to determine alkaloid yield. In addition, the alkaloid existing in the culture medium is determined by the same procedure. The total yield of alkaloid is listed in Table 5.

Comparative example 16

Example 23 is repeated without the addition of gibberollin $GA_3$ to the culture medium. The total yield of alkaloid is exhibited in Table 5.

Table 5

| | Amount of $GA_3$ addition (mol concentration) | Total yield of alkaloid | |
|---|---|---|---|
| | | Hyoscyamine | Scopolamine |
| Comparative example 16 | 0 | 352 | 985 |
| Example 23 | $10^{-5}$ | 416 | 1218 |

Examples 24 - 26

Liquid Nitsch Nitsch culture medium of 20 mℓ containing $10^{-5}$M indolebutyric acid is fed in a 100 mℓ Erlenmeyes flask, and the seeds of the adventitious roots of Duboisia in 0.5g used in the above Example 23 are transplanted and cultured in the culture medium. The culture is carried out with the addition of $GA_3$ in concentrations of $10^{6}$M, $10^{-5}$M or $10^{-4}$M to the culture medium after three weeks from the commencement of culture instead of the addition at the start of culture as is the case with Example 23. After further one (1) week, the liquid culture medium alone is totally recovered from the flask, and the alkaloid in the culture medium is determined by gas chromatography. The result is shown in Table 6 as Run 1.

Then, new Nitsch Nitsch liquid culture-medium of 20 m is added to the culture flask not containing the above liquid culture medium. After 3 weeks of culture, $GA_3$ is added in the same manner as above so as to cause a concentration of $10^{-6}$M, $10^{-5}$M or $10^{-4}$M to continue culturing. After further one (1) week, the liquid culture medium alone is totally recovered from the culture flask, and the alkaloid contained in the medium is determined. The result is exhibited in Table 6 as Run 2.

Comparative example 17

According to the conventional procedure, 0.5 g of seed cell of adventitious roots of Duboisia is fed in Nitsch Nitsch liquid culture medium (20 mℓ) containing $10^{-5}$M indolebutyric acid using 100 mℓ Erlenmeyer flask, and cultured for 4 weeks. After stopping culture, the adventitious roots and culture medium are recovered. The adventitious roots thus recovered contain 396 µg of hyoscyamine and 1023 µg of scopolamine, while those existing in the culture medium are 2 µg and 5 µg, respectively.

Table 6

| Experiment | Gibberellin Mol concentration | Run | Amount of alkaloid released in culture medium (µg/20 ℓ culture medium) | |
|---|---|---|---|---|
| | | | Hyoscyamine | Scopolamine |
| Comparative example 17 | None | 1 | 2 | 5 |
| Example 24 | $GA_3$ $10^{-6}$ | 1 | 53 | 186 |
| | | 2 | 42 | 132 |
| Example 25 | $GA_3$ $10^{-5}$ | 1 | 173 | 518 |
| | | 2 | 136 | 438 |
| Example 26 | $GA_3$ $10^{-4}$ | 1 | 238 | 725 |
| | | 2 | 89 | 235 |

Example 27

Example 1 is repeated with addition of abscisic acid to a concentration of $10^{-7}$M instead of spermidine. The result is indicated in Table 7.

Comparative example 18

Example 27 is repeated without addition of abscisic acid to the culture medium. The yield of alkaloid thus obtained is also shown in Table 7.

Example 28

Example 27 is repeated with addition of 4-butylamine-2-methylthiopyrido [2,3-d] pyridine, one of anti-cytokinin substances, to a concentration of $10^{-6}$M instead of abscisic acid. The yield of alkaloid thus obtained is also listed in Table 7.

Table 7

| | Substance added | Amount of growth (g/ℓ) | Alkaloid content (%) | |
|---|---|---|---|---|
| | | | Scopolamine | Hyoscyamine |
| Comparative example 18 | --- | 9.5 | 0.60 | 0.20 |
| Example 27 | Abscisic acid $10^{-7}$M | 9.0 | 0.95 | 0.25 |
| Example 28 | BAMP $10^{-6}$M | 9.4 | 0.90 | 0.26 |

BAMP: 4-butylamino-2-methylthiopyrido [2,3-d] pyrimidine

Availability in Industry

The adoption of the method for manufacturing tropane alkaloid by tissue culture according to the present invention enables efficient mass-production of tropane alkaloid, particularly scopolamine and/or hyoscyamine as compared with the conventional methods.

## Claims

1. A method for manufacturing tropane alkaloid characterized in that tropane alkaloid is manufactured by subjecting the tissues or cells of a plant which produces tropane alkaloid to tissue culture by use of a culture medium containing at least one compound selected from the group consisting of (A) amines; (B) sulfur-containing amino acids and/or oxyamino acids; (C) gibberellin; and (D) abscisic acid and/or antioytokinin substances.

2. The method as set forth in claim 1 characterized in that the concentration of said sulfur-containing amino acids and/or oxyamino acids in said culture medium is in the range of 0.3 to 10 mM.

# INTERNATIONAL SEARCH REPORT

International Application No PCT/JP88/00325

**I. CLASSIFICATION OF SUBJECT MATTER** (if several classification symbols apply, indicate all)[6]

According to International Patent Classification (IPC) or to both National Classification and IPC

Int.Cl[4] C12P17/18, C12N5/00// (C12P17/18, C12R1:91)

**II. FIELDS SEARCHED**

Minimum Documentation Searched[7]

| Classification System | Classification Symbols |
|---|---|
| IPC | C12P17/18, C12N5/00, C12R1:91 |

Documentation Searched other than Minimum Documentation to the Extent that such Documents are Included in the Fields Searched[8]

CAS, BIOSIS

**III. DOCUMENTS CONSIDERED TO BE RELEVANT**[9]

| Category*[10] | Citation of Document,[11] with indication, where appropriate, of the relevant passages[12] | Relevant to Claim No.[13] |
|---|---|---|
| A | J. Plant Physiol., Vol.124, Nos. 1 to 2, (1986), T. Hashimoto et al., [Tropane Alkaloid Production in Hyoscyamus Root Cultures], P.61-76 | 1-2 |
| A | Agric. Biol. Chem., Vol.51, No.10, (1987) T. Hashimoto et al., [Effects of Culture Conditions on Tropane Alkaloid Formation in Hyoscyamus nigar Suspention Cultures], P.2769-2774 | 1-2 |
| A | JP, A, 60-259196 (Sekisui Plastics Co., Ltd.), 21 December 1985 (21. 12. 85) (Family: none) | 1-2 |
| A | JP, A, 62-32880 (Seitaikinou Riyou Kagakuhin Shinseizougijutsu Kenkyu Kumiai), 12 February 1987 (12. 02. 87) (Family: none) | 1-2 |

* Special categories of cited documents:[10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| July 4, 1988 (04. 07. 88) | July 11, 1988 (11. 07. 88) |
| **International Searching Authority** | **Signature of Authorized Officer** |
| Japanese Patent Office | |